(19) Europäisches Patentamt
European Patent Office
Office européen des brevets

(11) EP 1 196 590 B1

(12) **EUROPÄISCHE PATENTSCHRIFT**

(45) Veröffentlichungstag und Bekanntmachung des Hinweises auf die Patenterteilung:
**09.07.2008 Patentblatt 2008/28**

(21) Anmeldenummer: **00945955.3**

(22) Anmeldetag: **26.07.2000**

(51) Int Cl.:
*C12N 15/31* (2006.01) *C12N 9/14* (2006.01)
*C12P 41/00* (2006.01) *C12Q 1/34* (2006.01)

(86) Internationale Anmeldenummer:
**PCT/EP2000/007211**

(87) Internationale Veröffentlichungsnummer:
**WO 2001/007623 (01.02.2001 Gazette 2001/05)**

(54) **VERFAHREN ZUR TRENNUNG VON EPOXID-ENANTIOMERGEMISCHEN MITTELS EPOXIDHYDROLASEPRODUZIERENDEN MIKROORGANISMEN**

PROCESS OF SEPARATION OF EPOXIDE-ENANTIOMER MIXTURES BY EPOXIDE-HYDROLASE PRODUCING MICROORGANISMS

PROCÉDÉ DE SÉPARATION D'ÉNANTIOMÈRES D'ÉPOXYDES À L'AIDE DE MICROORGANISMES PRODUISANT L'ÉPOXYDE HYDROLASE

(84) Benannte Vertragsstaaten:
**AT BE CH CY DE DK ES FI FR GB GR IE IT LI LU MC NL PT SE**

(30) Priorität: **27.07.1999 DE 19935113**

(43) Veröffentlichungstag der Anmeldung:
**17.04.2002 Patentblatt 2002/16**

(73) Patentinhaber: **BASF SE**
**67056 Ludwigshafen (DE)**

(72) Erfinder:
- **ZOCHER, Frank**
**D-70569 Stuttgart (DE)**
- **ENZELBERGER, Markus**
**D-70569 Stuttgart (DE)**
- **SCHMID, Rolf, D.**
**D-70569 Stuttgart (DE)**
- **WOHLLEBEN, Wolfgang**
**D-72076 Tübingen (DE)**
- **HAUER, Bernhard**
**D-67056 Fussgönheim (DE)**

(74) Vertreter: **Kinzebach, Werner et al**
**Patent Attorneys**
**Reitstötter, Kinzebach & Partner**
**Sternwartstrasse 4**
**81679 München (DE)**

(56) Entgegenhaltungen:
**WO-A-98/53081**

- **LUTZ-WAHL SABINE ET AL: "Stereo- and regioselective hydroxylation of alpha-ionone by Streptomyces strains." APPLIED AND ENVIRONMENTAL MICROBIOLOGY, Bd. 64, Nr. 10, Oktober 1998 (1998-10), Seiten 3878-3881, XP000946739 ISSN: 0099-2240**
- **ZOCHER FRANK ET AL: "A colorimetric assay suitable for screening epoxide hydrolase activity." ANALYTICA CHIMICA ACTA, Bd. 391, Nr. 3, 4. Juni 1999 (1999-06-04), Seiten 345-351, XP000946717 ISSN: 0003-2670**
- **RINK R ET AL: "PRIMARY STRUCTURE AND CATALYTIC MECHANISM OF THE EPOXIDE HYDROLASE FROM AGROBACTERIUM RADIOBACTER AD1" JOURNAL OF BIOLOGICAL CHEMISTRY,US,AMERICAN SOCIETY OF BIOLOGICAL CHEMISTS, BALTIMORE, MD, Bd. 272, Nr. 23, 6. Juni 1997 (1997-06-06), Seiten 14650-14657, XP002063249 ISSN: 0021-9258**
- **ARCHELAS A ET AL: "Epoxide hydrolases: new tools for the synthesis of fine organic chemicals" TRENDS IN BIOTECHNOLOGY,GB,ELSEVIER PUBLICATIONS, CAMBRIDGE, Bd. 16, Nr. 3, 1. März 1998 (1998-03-01), Seiten 108-116, XP004108588 ISSN: 0167-7799**
- **MISCHITZ M ET AL: "Asymmetric Microbial Hydrolysis of Epoxides" TETRAHEDRON: ASYMMETRY,NL,ELSEVIER SCIENCE PUBLISHERS, AMSTERDAM, Bd. 6, Nr. 6, 1. Juni 1995 (1995-06-01), Seiten 1261-1272, XP004048169 ISSN: 0957-4166**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- ARCHER I V J: "Epoxide Hydrolases as Asymmetric Catalysts" TETRAHEDRON,NL, ELSEVIER SCIENCE PUBLISHERS, AMSTERDAM, Bd. 53, Nr. 46, 17. November 1997 (1997-11-17), Seiten 15617-15662, XP004106413 ISSN: 0040-4020
- WEIJERS CAREL A G M ET AL: "Epoxide hydrolases from yeast and other sources: versatile tools in biocatalysis." JOURNAL OF MOLECULAR CATALYSIS B ENZYMATIC, Bd. 6, Nr. 3, 11. März 1998 (1998-03-11), Seiten 199-214, XP000946716 ISSN: 1381-1177
- ZOCHER F ET AL: "Epoxide hydrolase activity of Streptomyces strains" JOURNAL OF BIOTECHNOLOGY,NL,ELSEVIER SCIENCE PUBLISHERS, AMSTERDAM, Bd. 77, Nr. 2-3, Februar 2000 (2000-02), Seiten 287-292, XP004185827 ISSN: 0168-1656

## Beschreibung

Die Erfindung betrifft ein neues Verfahren zur enzymatischen Trennung von Epoxid-Enantiomerengemischen, ein neuartiges Nachweisverfahren für Epoxidhydrolaseaktivität, ein Screening-Verfahren zum Nachweis von Epoxidhydrolaseaktivität und die Verwendung von Bakterien der Gattung Streptomyces sp. und der daraus gewonnenen Epoxidhydrolasen zur enantioselektiven Epoxidhydrolyse.

Die zunehmende Bedeutung enantiomerenreiner Verbindungen vor allem in der pharmazeutischen und der agrochemischen Industrie erfordert einen sicheren und wirtschaftlichen Zugang zu optisch aktiven Substanzen. Für die Darstellung von enantiomerenreinen Diolen und Epoxiden steht eine Reihe von Methoden zur Verfügung:

Bei der asymmetrischen chemischen Synthese von Epoxiden geht man von einer prochiralen Verbindung aus. Durch Verwendung eines chiralen Reagens, beispielsweise einer chiralen Persäure, eines chiralen Dioxirans bzw. Oxaziridins oder eines chiralen Borats, eines chiralen Auxiliars oder eines chiralen, metallischen oder nicht-metallischen, Katalysators wird ein chirales Epoxid gebildet. Der bekannteste Weg zur Darstellung chiraler Epoxide ist die Sharpless-Epoxidierung von Alkenolen, z.B. von Allylalkoholen, mit Hydroperoxiden in Gegenwart von Übergangsmetallkatalysatoren.

Auch die Herstellung von enantiomerenreinen Diolen auf biochemischem Weg ist bekannt. Mikroorganismen mit Epoxidhydrolaseaktivität katalysieren die regio- und enantiospezifische Hydrolyse von Epoxiden. Sie spalten die Etherbindung in Epoxiden unter Bildung von Diolen. Es wurden bereits einige Bakterienstämme beschrieben, die es ermöglichen, eine breite Auswahl an racemischen Epoxiden enantioselektiv zu hydrolysieren. Die Zahl der bekannten Epoxidhydrolasen und ihre Anwendung für die organische Synthese ist jedoch bislang begrenzt. Bekannte Stämme mit Epoxidhydrolaseaktivität sind beispielsweise Aspergillus niger LCP521, Bacillus sulfurescens ATCC 7159, Rhodococcus species NCIMB 11216 und andere.

Die bekannten Stämme mit Epoxidhydrolaseaktivität weisen jedoch oft ein begrenztes Substratspektrum und geringe Reaktionsgeschwindigkeiten auf. Auch sind die mit diesen Stämmen erreichbaren Enantioselektivitäten vielfach zu gering [Grogan, G., et al., FEMS Microbiology Lett. 141 (1996), 239 - 243; Kroutil, W., et al., Tetrahedron Lett. (1996), 8379 - 8382]. Die bekannten Stämme sind genetisch schlecht zugänglich und teilweise schwierig kultivierbar. Deshalb liegen bisher nur zwei Epoxidhydrolasen [Corynebacterium sp. C12, (Misawa, E., et al., Eur. J. Biochem. 253 (1998), 173 - 183) und Agrobacterium radiobacter AD1 (Rink, R., et al., J.Biol.Chem. 272 (1997), 14650 - 14657)] rekombinant in E.coli vor. Auch die Reinigung der aus den Mikroorganismen erhaltenen Epoxidhydrolasen wurde bisher nur bei Rhodococcus species NCIMB 11216 [Faber, K., et al., Biotechnology Lett. 17 (1995), 893 - 898] und Norcardia EH 1 [Kroutil, W., et al J. Biotechnol. 61 (1998), 143 - 150] beschrieben. Sie war in beiden Fällen sehr aufwendig. Die Anreicherung von Epoxidhydrolase aus Corynebacterium sp. C12 wird von Misawa, E., et al., Eur. J. Biochem. 253,(1998) 173-183 beschrieben.

Zusätzlich erschwert wird die Suche nach neuen epoxidhydrolase-haltigen Mikroorganismen dadurch, daß das Screening, d.h. die systematische Suche, nach neuen Epoxidhydrolase produzierenden Mikroorganismen, z.B. in mikrobiologischen Stammsammlungen, bisher sehr zeitaufwendig war. Dies liegt daran, daß zum Screening üblicherweise Verfahren eingesetzt werden, bei denen die einzelnen Ansätze aufgearbeitet und einzeln gas- bzw. flüssigkeitschromatographisch untersucht werden müssen.

Eine erste Aufgabe der Erfindung war es daher, neue Epoxidhydrolasen mit erweitertem Substratspektrum und/oder verbesserter Reaktivität und/oder verbesserter Enantioselektivität bereitzustellen. Außerdem sollten die neuen Epoxidhydrolasen insbesondere dadurch besser zugänglich sein, daß sie aus nicht-pathogenen, leicht kultivierbaren Organismen isolierbar sind und außerdem gegebenenfalls eine gute molekularbiologische Zugänglichkeit aufweisen.

Eine zweite Aufgabe der Erfindung war die Bereitstellung eines Verfahrens zum schnelleren und einfacheren Nachweis von Epoxidhydrolase, was auch ein verbessertes Screening auf Epoxidhydrolase produzierende Mikroorganismen ermöglichen sollte.

Eine dritte Aufgabe der Erfindung war die Bereitstellung eines verbesserten biochemischen Verfahrens zur Trennung von Epoxid-Enantiomerengemischen und damit eines verbesserten Verfahrens zur enantioselektiven Umsetzung von Epoxiden, das einen einfacheren Zugang zur enantiomerenreinen Diolen und/oder Epoxiden ermöglicht.

Eine vierte Aufgabe der Erfindung bestand darin, neue Epoxidhydrolase produzierende Mikroorganismen bereitzustellen.

Die vorliegende Erfindung basiert unter anderen auf der Verwendung von Epoxidhydrolasen (E.C. 3.3.2.3) aus Mikroorganismen der Gattung Streptomyces sp. Epoxidhydrolase-Aktivität wurde in Mikroorganismen dieser Gattung bisher nicht beschrieben.

Die erfindungsgemäß Verwendeten Epoxidhydrolasen besitzen wenigstens eine der folgenden vorteilhaften Eigenschaften im Vergleich zu bisher bekannten Epoxidhydrolasen:

- verbesserte Enantioselektivität bei der Spaltung von enantiomerer Epoxide;
- verbessertes (erweitertes) Substratspektrum;

- verbesserte Reaktivität;
- bessere molekularbiologische Zugänglichkeit;
- bessere biochemische Zugänglichkeit aufgrund leichterer Kultivierbarkeit der Mikroorganismen.

Eine "verbesserte Enantioselektivität" im Sinne der Erfindung ist gegeben, wenn bei im wesentlichen gleichem Umsatz ein höherer Enantiomerenüberschuß erzielbar ist.

Ein "erweitertes Substratspektrum" in Sinne der Erfindung bedeutet, daß racemische Gemische mehrerer Epoxide umgesetzt werden.

Eine "verbesserte Reaktivität" im Sinne der Erfindung bedeutet, daß die Umsetzung mit höherer Raum-Zeit-Ausbeute erfolgt.

Die Erfindung basiert insbesondere auf solchen Epoxidhydrolasen aus Streptomyces, die wenigstens eine der folgenden Eigenschaften aufweisen:

a) hydrolytische Epoxidspaltung eines Styroloxids, wie z.B. Styroloxid oder eines am Phenylring oder Epoxidring ein- oder mehrfach substituierten Derivats davon, wie insbesondere einem in meta- oder para-Stellung durch Nitro oder Halogen, insbesondere Chlor oder Brom, monosubstituierten Styroloxids, und wenigstens einer weiteren Verbindung ausgewählt unter 3-Phenyl-ethylglycidat, n-Hexan-1,2-oxid, n-Decan-1,2-oxid und Indenoxid, welche gegebenenfalls ein- oder mehrfach durch Substituenten, vorzugsweise gemäß obiger Definition, substituiert sein können;

b) Umsetzung eines Racemats von Styroloxid mit einer Enantioselktivität E ≥ 2, wie z.B. ≥ 10, wie etwa 10 bis 100, zu (S)-Phenyl-1,2-ethanol gemäß der im folgenden angegebenen Reaktionsgleichung (A), wobei diese Umsetzung mit ganzen Zellen oder einem Zellhomogenat oder einem angereicherten oder gereinigten Enzympräparat durchführbar ist und bevorzugt in Gegenwart eines Cosolvens, wie 5 bis 10% (v/v) DMSO erfolgt.

Gemäß einer bevorzugten Ausführungsform werden Epoxidhydrolasen eingesetzt, welche aus Bakterien der Gattung Streptomyces sp., insbesondere aus der Art S. griseus, S. thermovulgaris, S. antibioticus, S. arenae und S. fradiae, vorzugsweise aus den Stämmen Streptomyces griseus (DSM 40236 und DSM 13447), Streptomyces thermovulgaris (DSM 40444 und DSM 13448), Steptomyces arenae Tü 495 (DSM 40737 und DSM 12134) Streptomyces antibioticus Tü 4 (DSM 12925) oder Streptomyces fradiae Tü 27 (DSM 12131) isolierbar sind.

Besonders bevorzugt ist die aus Streptomyces antibioticus Tü 4 (DSM 12925) isolierbare Epoxidhydrolase. Dieses Enzym ist durch seine ausgeprägte Enantioselektivität bei der Umsetzung von (R/S)-Styroloxid (I) gemäß folgender Reaktionsgleichung (A)

Reaktionsgleichnung (A):

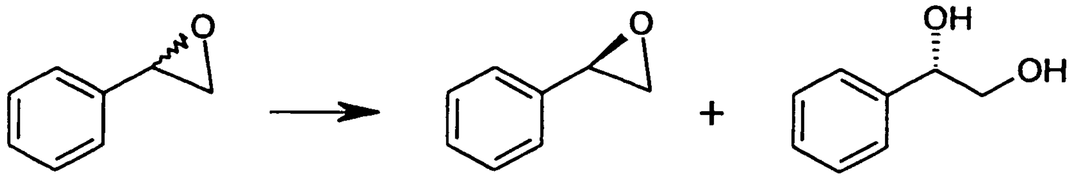

(I) (II) (III)

zu (S)-Phenyl-1,2-ethandiol (III) unter Nicht-Hydrolyse von (R)-Styroloxid (II) charakterisiert. So katalysiert dieses Enzym in einem Reaktionsmedium, enthaltend 10 %(v/v) DMSO als Lösungsvermittler, obige Umsetzung unter einer Enantioselektivität von E = 13 und einem Enantiomerenüberschuß ee[%] = 99 für (II) und ee[%] = 14 für (III).

Die Isolierung des Enzyms wird in den Ausführungsbeispielen näher beschrieben. Soweit keine näheren Angaben gemacht werden, erfolgt die Enzym-Anreicherung mit Hilfe biochemischer Standardverfahren, wie z.B. beschrieben von T.G. Cooper in Biochemische Arbeitsmethoden, Verlag Walter de Gruyter, Berlin, New York, (1981). Beispielsweise eignen sich Reinigungsverfahren, wie Fällung, z. B. mit Ammoniumsulfat, Ionenaustauschchromatographie, Gelchromatographie, Affinitätschromatographie, z. B. Immunoaffinitätschromatographie und isoelektrische Fokussierung, sowie Kombinationen solcher Verfahren.

Die Erfindung basiert auch auf der Verwendung des Streptomyces-Stamms mit der Bezeichnung Streptomyces antibioticus Tü 4, hinterlegt bei der DSMZ unter der Hinterlegungsnummer DSM 12925 und Varianten und Mutanten dieses Stammes.

Gegenstand der Erfindung sind ebenfalls die Verwendung funktionaler Analoga der oben erwähnten Enzyme,

wie Varianten, Allele und Mutanten, welche Epoxydhydrolase-Aktivität besitzen und, vorzugsweise, wenigstens eine der obengenannten vorteilhaften Eigenschaften aufweisen.

Obige zweite Aufgabe konnte überraschenderweise gelöst werden durch Bereitstellung eines optischen Nachweisverfahrens für Epoxidhydrolase, wobei man

a) einen Analyten, z.B. eine Mikroorganismenkultur, in welchen man Epoxidhydrolaseaktivität vermutet, mit einem epoxidhaltigen Substrat für die Epoxidhydrolase unter Reaktionsbedingungen inkubiert;

b) nicht umgesetztes Epoxid mit 4-Nitrobenzylpyridin (NBP) unter Bildung eines bei 560nm absorbierenden Farbstoffs chemisch umsetzt; und

c) den Ansatz aus Schritt b) auf Abnahme der Farbstoffkonzentration, relativ zu einem Epoxidhydrolase-freien Kontrollansatz analysiert.

Das erfindungsgemäße Nachweisverfahren ist qualitativ, z.B. als Spottest, oder quantitativ durchführbar. Beim quantitativen Verfahren bestimmt man zunächst quantitativ die relative Abnahme der Farbstoffkonzentration, z.B. photometrisch durch Bestimmung der Absorption bei 560 nm, und ermittelt daraus die Epoxidhydrolaseaktiviät im Analyten.

Als Analyt sind grundsätzlich geeignet Mikroorganismen per se, wie z.B. Proben aus einer frisch gezogenen Kultur eines Bakteriums, Zellhomogenisate davon oder Fraktionen dieser Zellhomogenisate nach einer Aufreinigung. Vorzugsweise wird der Test mit ganzen Zellen oder nach Aufschluß der Zellen, z.B. mit Ultraschall oder Lysozym, durchgeführt.

Beispielsweise ist das Verfahren anwendbar auf den Nachweis von Epoxidhydrolase in Bakterien aus der Gattung Streptomyces.

Vorzugsweise wird das Nachweisverfahren so durchgeführt, daß man einer frisch gezogenen Kultur des Mikroorganismus eine Probe entnimmt, diese aufschließt, von Zellfragmenten befreit und mit einem Epoxid, welches ein Substrat für das zu testende Enzym unfasst, zugibt und mischt. Erforderlichenfalls kann man in dem Ansatz die Reaktionsbedingungen durch übliche Maßnahmen, wie z.B. durch Zugabe von Puffer, Einstellen der Reaktionstemperatur und dergleichen, optimieren. Bevorzugt verwendet man zur Verbesserung der Löslichkeit des Epoxids im wässerigen Reaktionsmedium ein Cosolvens. Geeignete Cosolventien sind z.B. Dimethylsulfoxid (DMSO), Dimethylformamid (DMF), Ethanol oder Aceton. Optimale Reaktionsbedingungen für Epoxidhydrolasen aus Streptomyces umfassen:

| | |
|---|---|
| Reaktionsmedium: | Natriumphosphatpuffer pH 8,0, 0,1 M, 10% (v/v) DMSO |
| pH-Bereich: | 6-8 |
| Temperatur: | 30-37 °C |
| Reaktionsdauer: | 2-20 Stunden |
| Substratkonzentration: | 0,1 bis 0,8 molar |

Vorzugsweise verwendet man als Substrat Styroloxid, 3-Phenylglycidat, Hexan-1,2-oxid, Decan-1,2-oxid und/oder Indenoxid in enantiomerenreiner Form oder als Enantiomerengemisch.

Zur Entwicklung der Farbreaktion wird anschließend der pH-Wert durch Zugabe einer Base, wie z.B. Triethylamin, eingestellt. Anschließend gibt man NBP, z.B. in einer Konzentration im Bereich von etwa 3 bis 10 , vorzugsweise etwa 5 % (w/v) in Methoxyethanol, hinzu. Als Lösungsvermittler für den gebildeten Farbstoff gibt man z.B. Triethylenglycoldimethylether in ausreichender Menge hinzu. Den Ansatz inkubiert man schließlich bei etwa 35 bis 45, vorzugsweise 39°C und bestimmt anschließend die Absorption bei 560 nm, vorzugsweise gegen eine Referenz bei 650 nm. Aus einem Vergleich mit einem enzymfreien Kontrollansatz kann man die Abnahme der Absorption und damit die Abnahme von Epoxid quantitativ bestimmen.

Gegenstand der Erfindung sind auch Screening-Verfahren zum Nachweis von Mikroorganismen mit Epoxidhydrolaseaktivität und/oder mit der Befähigung zur enantioselektiven Hydrolyse von Epoxiden, umfassend das oben beschriebene Nachweisverfahren. Dieses eignet sich besonders zur systematischen Untersuchung von Stammsammlungen oder durch sog. "directed evolution" erzeugte Mutantenbanken auf Epoxidhydrolaseaktivität.

Obige dritte Aufgabe konnte überraschenderweise gelöst werden durch Bereitstellung eines Verfahrens zur Trennung von Epoxid-Enantiomerengemischen, das dadurch gekennzeichnet ist, daß man

a) ein Epoxid-Enantiomerengemisch, welches ein Epoxydhydrolase-Substrat umfasst, mit einer erfindungsgemäßen Epoxydhydrolase, einem Mikroorganismus der Gattung Streptomyces sp., einem Epoxidhydrolase-haltigen Homogenat davon oder einer Fraktion dieses Homogenates unter Reaktionsbedingungen inkubiert;

b) das Enantiomerengemisch, vorzugsweise bis zum Erreichen von 50 %-igem Umsatz, umsetzt; und

c) das im Reaktionsgemisch verbleibende Enantiomer vom Umsetzungsprodukt trennt und das im wesentlichen enantiomerenreine umsetzungsprodukt und/oder das verbleibende im wesentlichen enantiomerenreine Edukt reinigt.

Vorzugsweise setzt man Enantiomerengemische eines der folgenden Epoxide um: Styroloxid, 3-Phenylglycidat, He-xan-1,2-oxid, Decan-1,2-oxid und Indenoxid oder substituierte Analoga dieser Oxide gemäß obiger Definition.

Ein weiterer Gegenstand der Erfindung betrifft ein Verfahren zur Gewinnung von Epoxydhydrolasen (E.C. 3.3.2.3), dadurch gekennzeichnet, daß man

a) aus einer Kultur eines Mikroorganismus der Gattung Streptomyces sp. ein Zellhomogenat herstellt;

b) das Homogenat fraktioniert, wobei man die erhaltenen Fraktionen auf Epoxidhydrolase-Aktivität, vorzugsweise unter Anwendung eine Nachweisverfahren basierend auf der Farbreaktion von unumgesetztem Epoxid mit NBP gemäß obiger Definition, testet; und

c) Fraktionen mit Epoxidhydrolase-Aktivität vereinigt und gegebenenfalls weiter fraktioniert.

Ein letzter Gegenstand der Erfindung betrifft die Verwendung einer erfindungsgemäßen Epoxidhydrolase, eines Mikroorganismus der Gattung Streptomyces sp., eines Epoxidhydrolase-haltigen Homogenates davon oder einer Fraktion dieses Homogenates zur enantioselektiven Hydrolyse von Epoxiden.

Die Erfindung wird durch die nachfolgenden Beispiele und unter Bezugnahme auf die beiliegenden Figuren näher erläutert. Dabei zeigt:

Figur 1 Meßergebnisse für die Farbstoffbildung aus NBP und Epoxid bei verschiedenen Styroloxidkonzentrationen. Aufgetragen ist die Absorptionsänderung in Ansätzen in Gegenwart von E. coli DH5a nach dem NBP-Assay (schwarze Kreise), ohne E. coli DH5a-Zelle und ohne NBP (schwarze Quadrate) und mit Zelllysat von E. coli DH5a nach NBP-Assay (schwarze Dreiecke), jeweils nach 45 Minuten bei den angegebenen Styroloxidkonzentrationen.

Figur 2 den zeitlichen Verlauf der Racematspaltung von Styroloxid durch die Epoxidhydrolase aus S. antibioticus Tü4 (DSM 12925). ee[%] (R)-Styroloxid (schwarze Quadrate); ee[%] (S)-Phenyl-1,2-ethandiol (schwarze Kreise); Umsatz[%] (schwarze Dreiecke);

Figur 3 den Einfluß verschiedener Cosolventien auf die Hydrolyse von Styroloxid durch Epoxidhydrolase aus S. antibioticus Tü4 (DSM 12925); aufgetragen ist ee[%] (R)-Styroloxid in Abhängigkeit von der Cosolvenskonzentration (%(v/v)); Aceton (schwarze Quadrate); DMSO (schwarze Kreise); DMF (schwarze Dreiecke).

Beispiel 1 : Validierung eines Testsystems für Epoxidhydrolase

a) Styroloxid als Substrat

Ein Epoxidhydrolase-freier Organismen (E. coli DH5a) wurden in 2 ml Kulturen kultiviert und aufgeschlossen. Jeweils 100 µl der erhaltenen Zellaufschlüsse wurden auf 4 Vertiefungen einer Mikrotiterplatte verteilt. Zu dem Zellaufschluß wurden 50 µl Aceton mit verschiedenen Styroloxidmengen (1,3 mol/l (Stammlösung), 0,65 mol/l, 0,32 mol/l, 0,16 mol/l, 0,08 mol/l) hinzugegeben. Die Lösung wurde 2 Stunden bei 30°C inkubiert. Nach Zugabe von 25 µl Triethylamin (TE), 50 µl p-Nitrobenzylpyridin (NBP, 5 Vol.-%) und 50 µl Triethylenglykoldimethylether wurde nochmals 45 Minuten bei 39°C inkubiert und anschließend die Absorption bei 560 nm gegen eine Referenz bei 650 nm gemessen.

Die Ergebnisse sind in Figur 1 dargestellt. Die Absorption nahm linear zur eingesetzten Styroloxidmenge zu. Deshalb zeigt die Verringerung der Absorption das Vorhandensein einer Epoxidhydrolaseaktivität an.

b) 3-Phenylglycidat als Substrat

Beispiel 1a) wurde mit der Abänderung wiederholt, daß als Substrat 3-Phenylglycidat verwendet wurde. Auch hier fand sich eine Linearität zwischen der Absorption und der verbleibenden Menge an nicht hydrolysiertem Epoxid.

c) Hexan-1,2-oxid als Substrat

Beispiel 1a) wurde mit der Abänderung wiederholt, daß als Substrat Hexan-1,2-oxid verwendet wurde. Auch hier fand sich eine Linearität zwischen der Absorption und der verbleibenden Menge an nicht hydrolysiertem Epoxid.

d) Indenoxid als Substrat

Beispiel 1a) wurde mit der Abänderung wiederholt, daß als Substrat Indenoxid verwendet wurde. Auch hier fand sich eine Linearität zwischen der Absorption und der verbleibenden Menge an nicht hydrolysiertem Epoxid.

Beispiel 2: Screening verschiedener Streptomyces-Stämme auf Epoxidhydrolase-Aktivität mit Styroloxid als Substrat

Analog zu Beispiel 1 wurden verschiedene hinterlegte Streptomyces-Stämme sowie als negative Kontrolle ein Bakterienstamm der Gattung Rhodococcus sp. (NCIMB 11216) getestet. Diejenigen Vertiefungen der Mikrotiterplatte welche die negative Kontrolle enthielten zeigten eine intensive Farbreaktion (Blaufärbung) während in Vertiefungen mit Streptomyces antibioticus Tü4 (DSM 12925) und Streptomyces fradiae Tü27 (DSM 12131) die höchste Epoxidhydrolase-Aktivität nachweisbar war (Ergebnisse nicht gezeigt).

Beispiel 3: Kultivierung des Stammes Streptomyces antibioticus Tü4

Die Kultivierungen wurden sowohl in 250 ml Schüttelkolben als auch in einem 30 1 Fermenter durchgeführt, wobei der pH-Wert nicht kontrolliert wurde.

a) 250 ml Fermentation: Die Kultivierungsdauer betrug im 250 ml Maßstab zwischen 48 und 72 Stunden bei 30°C.

b) 30 1 Fermentation: 2 Schüttelkolben mit je 1 1 Malzmedium [10 g Malzextrakt, 4 g Hefe, auf 1 1 mit Leitungswasser aufgefüllt und autoklaviert, sterilfiltrierte Glucose (4g/l Endkonzentration) wurde nach dem Autoklavieren hinzugegeben] wurden mit Sporen von Streptomyces antibioticus Tü4 angeimpft; anschließend wurde 48 Stunden geschüttelt (30°C, 210 U/min). Mit 2 1 dieser Vorkultur wurden 20 1 Malzmedium (Zugabe von 0,5 1 20 gew.-%ige Glucoselösung zu 20 1 Malzextrakt/Hefelösung) angeimpft, wobei sich für ein möglichst disperses Wachstum der Kolonie die vorherige Homogenisierung (Glashomogenisator, Braun Chemie) empfiehlt. Es wurde im 30 1-Fermenter bei 30°C 24 Stunden gerührt (17 l/min 02, 200 U/ min), dann wurde die Fermentation abgebrochen. Die Zellen wurden bei 4000 U/min 30 Minuten abzentrifugiert und mit TE-Puffer (pH 7,3, 1 mmol/l EDTA) gewaschen. Die Biofeuchtmasse betrug 318 g. Die Zellen wurden in 400 ml TE-Puffer (pH 7,3, mmol/l EDTA) resuspendiert.

Beispiel 4: Anreicherung Epoxidhydrolase aus S. antibioticus Tü4

a) Zellaufschluß:

Für die Versuche mit aufgeschlossenen Zellen wurden die kultivierten Zellen durch Filtration oder Zentrifugation abgetrennt und zweimal mit Phosphatpuffer (0,1 mol/l NaCl, 10 mmol/l EDTA) gewaschen. Anschließend wurde eine Suspension (10% w/v) im gleichen Puffer mit zusätzlich 10 mg/ml Lysozym hergestellt. Die Suspension wurde 1 Stunde bei 30°C inkubiert und anschließend zweimal je 5 Minuten, dazwischen 1 Minute Pause, im Eisbad mit Ultraschall aufgeschlossen (Branson Sonifier W250, Output 80W). Die erhaltene Lösung wurde 60 Minuten bei 32 500 x g abzentrifugiert und filtriert (0,22mm Sterivex-GP Filter, Millipore)

b) Aufarbeitung des Zellextraktes

Eine mit einem Anionenaustauscher gefüllte Säule (Super Q 650M, Toso Haas, 60 ml Volumen) wurde mit 25 ml Tris/HCl-Puffer (pH 8,1) äquilibriert, dann wurden 15 ml des Rohextraktes aufgebracht. Es wurde mit 2 mol/l NaCl-Lösung mit folgenden Gradienten eluiert:

1. Gradient: 30 ml NaCl-Lösung (0-12 Gew.-% ),
2. Gradient: 60 ml NaCl-Lösung (12 - 35 Gew.-%),

Spülung mit 25 ml NaCl-Lösung (100 Gew.-%), Reäquilibrierung mit 60 ml Tris/HCl-Puffer.
Durchlauf: 4 ml/min, Fraktionsgröße 4 ml. Die Proteinbestimmung wurde bei 280 nm durchgeführt.
Die Aktivität der einzelnen Fraktionen wurde mit dem NBP-Assay untersucht. Hierzu wurden 150 µl aus den

Fraktionen in die Vertiefungen einer 96er Mikrotiterplatte gegeben und der NBP-Assay mit dem Biomek 2000 Pipettierroboter wie zuvor beschrieben durchgeführt. Es wurden 50 µl Acetonlösung mit Styroloxid (2,6 mol/l) verwendet, um die Acetonmenge zu verkleinern, da die Zugabe von Aceton in einer relativ zu großen Menge zu einer Verringerung der Enzymaktivität führen kann. Die restliche Versuchsdurchführung erfolgte analog zu der vorstehend beschriebenen Vorgehensweise.
In der Anreicherung konnte in einer Fraktion die gesamte Epoxidhydrolaseaktivität detektiert werden.

Beispiel 5: Umsetzung von Styroloxid mit Epoxidhydrolase aus S. antibioticus Tü4

a) Durchführung der Reaktion

Zu 250 ml des Zellaufschlusses aus Beispiel 4 mit 12,5 ml DMSO als Lösungsvermittler wurden 500 µg Styroloxid gegeben. Es wurde bei 30°C und gleichmäßigem Schütteln (250 U/min) inkubiert.
Nach 24 Stunden wurde die Reaktion durch Zugabe von 30 ml Essigsäureethylester abgebrochen, und die wäßrige Phase wurde extrahiert. Die organische Phase wurde im Vakuum eingeengt. Die gaschromatographische Analyse ergab einen Enantiomerenüberschuß des Substrats von ($ee_s$[%]) von 100 und des Produkts ($ee_p$[%]) von 14.
Die Enantiomerenreinheit einer chiralen Substanz, welche in (R)-und in (S)-Form auftritt, wird über den Parameter ee (Enantiomerenüberschuß) ausgedrückt. Dieser ist durch folgende allgemeine Gleichung definiert:

$$ee[\%] = [(X_A - X_B)/(X_A + X_B)] * 100$$

worin $X_A$ und $X_B$ für den Molenbruch von Enantiomer A bzw. B stehen.
Die Analyse der Enantiomerenüberschüsse erfolgte durch chirale Gaschomatographie unter folgenden Bedingungen: 75°C isotherm, 130 kPa, auf einer FS-Cyclodex β-I/P CS-Fused Silica Kapillarsäule (CS-Chromatographie Service GmbH, Langerwehe) (H2 Trägergas, Split 1:100, 0,25 mm x 50 mm).
Die Analyse des Produkts Phenyl-1,2-ethandiol erfolgte unter folgenden Bedingungen: 140°C isotherm unter ansonsten gleichen Bedingungen.
Die Enantioselektivität E einer enzymatischen Reaktion ist eine von Substratkonzentration und Umsatz unabhängige Konstante für ein Enzym und bei irreversibler Reaktion ohne Produktinhibition nach folgender Formel zu berechnen:

$$\begin{aligned} E &= (V_{max}/K_m)_{(R)\text{-Enantiomer}} / (V_{max}/K_m)_{(S)\text{-Enantiomer}} = \\ &= \{\ln[(1-U)(1-ee_s)]\}/\{\ln[(1-U)(1+ee_s)]\} \\ &= \{\ln[(1-U)(1+ee_p)]\}/\{\ln[(1-U)(1-ee_p)]\} \end{aligned}$$

worin $ee_s$ der Enantiomerenüberschuß des Substrats und $ee_p$ der Enantiomerenüberschuß des Produkts ist und der Umsatz U nach der Formel $U = ee_s/(ee_s+ee_p)$ berechenbar ist.
Die Auswertung erfolgte gemäß Chen, C.S., et al., (1982) J.Am.Chem.Soc. 104, 7294.

b) Ermittlung der Enantioselektivität

Für die Verfolgung des Reaktionsverlaufs wurden 1,5 ml Ansätze des Zellaufschlusses mit 75 ml DMSO und 7 ml Styroloxid in verschraubbaren 2 ml Reaktionsgefäßen bei 30°C geschüttelt und nach vorgegebenen Zeiten mit 300 ml Essigsäureethylester extrahiert. Tabelle 1 zeigt die Ergebnisse.

Tabelle 1:

| Zeit [h] | $ee_s$%[%] | $ee_p$[%] | Umsatz [%] |
|---|---|---|---|
| 0,5 | - | - | - |
| 1 | 11 | 66 | 15 |
| 3,5 | 23 | 26 | 47 |

(fortgesetzt)

| Zeit [h] | $ee_s$%[%] | $ee_p$[%] | Umsatz [%] |
|---|---|---|---|
| 5 | 33 | 22 | 59 |
| 7 | 91 | 21 | 82 |
| 24 | 100 | 14 | 87 |

Der zeitliche Verlauf der Reaktion ist in beiliegender Figur 2 dargestellt.

Beispiel 6: Hydrolyse von Styroloxid durch die Streptomyces-Stämme S. fradiae Tü27 und S. arenae Tü495

Ganze Zellen einer 250 ml Kultur des Organismus wurden in 200 ml Natriumphosphatpuffer (0,1 M, pH 8) resuspendiert. Dazu gab man 500 µl Styroloxid und 10% (v/v) DMSO. Der Reaktionsansatz wurde bei 30°C mit 210 Upm in einem geschlossenen Erlenmeyerkolben geschüttelt. Proben (1,5 ml) wurden in verschiedenen Zeitintervallen zur Kontrolle des Reaktionsverlaufs gezogen, bei 14000 Upm 3 Minuten zentrifugiert und mit 300 µl Diethylether extrahiert. Die organischen Phasen wurden mit wasserfreien Natriumsulfat getrocknet und gaschromatographisch zur Bestimmung des Enantiomerenüberschusses, des Umsatzes und der Enantioselektivität wie oben beschrieben analysiert. Die Ergebnisse sind in folgender Tabelle 2 zusammengefaßt:

Tabelle 2:

| Stamm | Enantiomerenüberschuß | | Umsatz | Dauer |
|---|---|---|---|---|
| | $ee_s$[a] [%] | $ee_p$[b][%] | [%] | [h] |
| Tü27 | 70 | 23 | 75 | 48 |
| Tü495 | 52 | 38 | 60 | 24 |
| [a] (R)-Styroloxid<br>[b] (S)-Phenyl-1,2-ethandiol | | | | |

Beispiel 7: Einfluß von Cosolventien auf die Styroloxid-Hydrolyse.

Beispiel 6 wurde wiederholt, wobei jedoch S. antibioticus Tü4 als Mikroorganismus verwendet wurde, und DMSO, DMF bzw. Aceton in verschiedenen Mengen zu den Reaktionsansätzen gegeben wurden. Die Ergebnisse sind in Figur 3 dargestellt. 10 % DMSO, 5 % Acteon und 1-3 % DMF führten zu einer Zunahme des Enantiomerenüberschusses und damit der Enantioselektivität.

Beispiel 7: Hydrolyse von 3-Penylethylglycidat (3-PEG) und n-Decan-1,2-oxid durch Epoxidhydrolase aus S. antibioticus Tü4

Beispiel 5 wurde wiederholt, wobei anstelle von Styroloxid 3-PEG bzw. n-Decan-1,2-oxid als Substrat eingesetzt wurde.

a) Umsetzung von 3-PEG:

Im Unterschied zu Beispiel 5 erfolgte die gaschromatographische Analyse von 3-PEG bei 60kPa, 130°C (60 min), 180°C (5 min), 10 °C/min

b) Umsetzung von Decan-1,2-oxid

Das gebildete Decan-1,2-diol wurde mit Aceton und p-Toluolsulfonsäure als Katalysator in Ethylacetat derivatisiert. Isopropyliden-decan-1,2-diol wurde bei 120 °C unter den in Beispiel 5 angegebenen Bedingungen analysiert.

**Patentansprüche**

**1.** Verfahren zur Trennung von Epoxid-Enantiomerengemischen, **dadurch gekennzeichnet, daß** man

a) ein Epoxid-Enantiomerengemisch, welches ein Epoxidhydrolase-Substrat umfasst, mit einem eine Epoxidhydrolase (E.C. 3.3.2.3) produzierenden Mikroorganismus der Gattung Streptomyces sp., einem Epoxidhydrolase-haltigen Homogenat davon oder einer Fraktion dieses Homogenates inkubiert;
b) das Enantiomerengemisch, vorzugsweise bis zur Einstellung des Reaktionsgleichgewichtes, umsetzt; und
c) das Reaktionsgemisch auftrennt.

**2.** Verfahren nach Anspruch 1, **dadurch gekennzeichnet, daß** man ein Enantiomerengemisch eines Epoxids umsetzt, das ausgewählt ist unter Styroloxiden, 3-Phenylglycidaten, Hexan-1,2-oxiden, Decan-1,2-oxiden und Indenoxiden.

**3.** Verfahren nach Anspruch 1 oder 2, wobei der Mikroorganismus ein Bakterium der Gattung Streptomyces sp., insbesondere aus der Art S. griseus, S. thermovulgaris, S. antibioticus, S. arenae und S. fradiae, vorzugsweise aus den Stämmen Streptomyces griseus (DSM 13447), Streptomyces thermovulgaris (DSM 13448), Streptomyces arenae Tü495 (DSM 12134), Streptomyces antibioticus Tü 4 (DSM 12925) oder Streptomyces fradiae Tü 27 (DSM 12131), ist.

**4.** Verfahren nach einem der Ansprüche 1 bis 3, wobei die Epoxidhydrolase durch wenigstens eine der folgenden Eigenschaften **gekennzeichnet** ist:

a) hydrolytische Epoxidspaltung eines Styroloxids, und wenigstens einer weiteren Verbindung ausgewählt unter 3-Phenyl-ethylglycidaten, n-Hexan-1,2-oxiden, n-Decan-1,2-oxiden und Indenoxiden;
b) Umsetzung eines Racemats von Styroloxid mit einer Enantioeselektivität $E \geq 2$ zu (S)-Phenyl-1,2-ethanol.

**5.** Nachweisverfahren für Epoxidhydrolase, wobei man

a) einen Analyten, in welchen man Epoxidhydrolaseaktivität vermutet, mit einem epoxidhaltigen Substrat für die Epoxidhydrolase unter Reaktionsbedingungen inkubiert, wobei der Analyt ein Bakterium aus der Gattung Streptomyces sp., ein Homogenat davon oder eine Fraktion dieses Homogenates ist;
b) mit nicht umgesetztem Epoxid in Anwesenheit von 4-Nitrobenzylpyridin (NBP) eine Farbreaktion durchführt; und
c) den Ansatz aus Schritt b) auf Abnahme der Farbstoffkonzentration, relativ zu einem Epoxidhydrolase-freien Kontrollansatz analysiert.

**6.** Verfahren nach Anspruch 5, **dadurch gekennzeichnet, daß** man die relative Abnahme der Farbstoffkonzentration quantitativ bestimmt und daraus die Epoxidhydrolaseaktiviät im Analyten ermittelt.

**7.** Verfahren nach einem der Ansprüche 5 und 6, **dadurch gekennzeichnet, daß** das epoxidhaltige Substrat Styroloxid, 3-Phenylglycidat, Hexan-1,2-oxid und/oder Indenoxid in enantiomerenreiner Form oder ein Enantiomerengemisch davon ist.

**8.** Screening-Verfahren zum Nachweis von Mikroorganismen mit Epoxidhydrolaseaktivität und/oder mit der Befähigung zur enantioselektiven Hydrolyse von Epoxiden, umfassend ein Nachweisverfahren nach einem der Ansprüche 5 bis 7.

**9.** Verwendung eines Mikroorganismus der Gattung Streptomyces sp., eines Epoxidhydrolase-haltigen Homogenates davon oder einer Fraktion dieses Homogenates zur enantioselektiven Hydrolyse von Epoxiden.

**10.** Verwendung eines Mikroorganismus der Gattung Streptomyces sp., eines Epoxidhydrolase-haltigen Homogenates davon oder einer Fraktion dieses Homogenates zur enantioselektiven Herstellung von Hydroxiden aus den korrespondierenden Epoxiden.

**11.** Verfahren zur Gewinnung einer Epoxydhydrolase (E.C. 3.3.2.3) enthaltenden Proteinfraktion, **dadurch gekennzeichnet, daß** man

a) aus einer Kultur eines Mikroorganismus der Gattung Streptomyces sp. ein Zellhomogenat herstellt;
b) das Homogenat fraktioniert, wobei man die erhaltenen Fraktionen auf Epoxidhydrolase-Aktivität, gegebenenfalls unter Anwendung eines Nachweisverfahrens gemäß einem der Ansprüche 5 bis 7, testet; und
c) Fraktionen mit Epoxidhydrolase-Aktivität vereinigt und gegebenenfalls weiter fraktioniert.

**12.** Verwendung eines Mikroorganismus der Gattung Streptomyces sp. zur Gewinnung einer Epoxidhydrolase (E.C.

3.3.2.3).

**13.** Verwendung nach Anspruch 12, wobei die Epoxidhydrolase durch wenigstens eine der folgenden Eigenschaften **gekennzeichnet** ist:

a) hydrolytische Epoxidspaltung eines Styroloxids, und wenigstens einer weiteren Verbindung ausgewählt unter 3-Phenyl-ethylglycidaten, n-Hexan-1,2-oxiden, n-Decan-1,2-oxiden und Indenoxiden;
b) Umsetzung eines Racemats von Styroloxid mit einer Enantioeselektivität E ≥ 2 zu (S)-Phenyl-1,2-ethanol.

**14.** Verwendung nach Anspruch 12 oder 13, wobei die Epoxidhydrolase aus Bakterien der Gattung Streptomyces sp., insbesondere aus der Art S. griseus, S. thermovulgaris, S. antibioticus, S. arenae und S. fradiae, vorzugsweise aus den Stämmen Streptomyces griseus (DSM 13447), Streptomyces thermovulgaris (DSM 13448), Streptomyces arenae Tü495 (DSM 12134) Streptomyces antibioticus Tü 4 (DSM 12925) oder Streptomyces fradiae Tü 27 (DSM 12131), gewonnen wird.

## Claims

**1.** A process for separating epoxide enantiomer mixtures, which comprises

a) incubating an epoxide enantiomer mixture, which comprises an epoxide hydrolase substrate, with a microorganism which produces an epoxide hydrolase (E.C. 3.3.2.3) and is of the genus Streptomyces, an epoxide hydrolase-containing homogenate thereof, or a fraction of this homogenate;
b) converting the enantiomer mixture, preferably until the reaction equilibrium is established; and
c) fractionating the reaction mixture.

**2.** The process according to claim 1, wherein an enantiomer mixture of an epoxide is converted, which mixture is selected from styrene oxides, 3-phenylglycidates, hexane-1,2-oxides, decane-1,2-oxides and indene oxides.

**3.** The process according to claim 1 or 2, the microorganism being a bacterium of the genus Streptomyces, in particular from the species S. griseus, S. thermovulgaris, S. antibioticus, S. arenae and S. fradiae, preferably from the strains Streptomyces griseus (DSM 13447), Streptomyces thermovulgaris (DSM 13448), Streptomyces arenae Tü495 (DSM 12134), Streptomyces antibioticus Tü4 (DSM 12925) or Streptomyces fradiae Tü 27 (DSM 12131).

**4.** The process according to one of claims 1 to 3, the epoxide hydrolase having at least one of the following properties:

a) hydrolytic epoxide cleavage of a styrene oxide and of at least one other compound selected from ethyl 3-phenylglycidates, n-hexane-1,2-oxides, n-decane-1,2-oxides and indene oxides;
b) conversion of a racemate of styrene oxide with an enantioselectivity E ≥ 2 to give (S)-phenyl-1,2-ethanol.

**5.** A detection method for epoxide hydrolase, which comprises

a) incubating an analyte in which epoxide hydrolase activity is suspected with an epoxide-containing substrate for the epoxide hydrolase under reaction conditions, the analyte being a bacterium of the genus Streptomyces, a homogenate thereof or a fraction of this homogenate;
b) carrying out a color reaction with unreacted epoxide in the presence of 4-nitro-benzylpyridine (NBP); and
c) analyzing the solution from step b) for decrease in pigment concentration, relative to an epoxide hydrolase-free control solution.

**6.** The method according to claim 5, wherein the relative decrease in pigment concentration is determined quantitatively and the epoxide hydrolase activity in the analyte is determined therefrom.

**7.** The method according to one of claims 5 and 6, wherein the epoxide-containing substrate is styrene oxide, 3-phenylglycidate, hexane-1,2-oxide and/or indene oxide in enantiomerically pure form or an enantiomer mixture thereof.

**8.** A screening method for detecting microorganisms having epoxide hydrolase activity and/or having the ability for the enantioselective hydrolysis of epoxides, comprising a detection method according to one of claims 5 to 7.

**9.** The use of a microorganism of the genus Streptomyces, an epoxide-hydrolase-containing homogenate thereof or a fraction of this homogenate for the enantioselective hydrolysis of epoxides.

**10.** The use of a microorganism of the genus Streptomyces, an epoxide-hydrolase-containing homogenate thereof or a fraction of this homogenate for the enantioselective preparation of hydroxides from the corresponding epoxides.

**11.** A process for producing a protein fraction which comprises epoxide hydrolase (E.C. 3.3.2.3), wherein

a) a cell homogenate is produced from a culture of a microorganism of the genus Streptomyces;
b) the homogenate is fractionated, the resultant fractions being tested for epoxide hydrolase activity, if appropriate using a detection method according to one of claims 5 to 7; and
c) fractions having epoxide hydrolase activity are combined and if appropriate further fractionated.

**12.** The use of a microorganism of the genus Streptomyces for obtaining an epoxide hydrolase (E.C. 3.3.2.3).

**13.** The use according to claim 12, the epoxide hydrolase having at least one of the following properties:

a) hydrolytic epoxide cleavage of a styrene oxide and of at least one other compound selected from ethyl 3-phenylglycidates, n-hexane-1,2-oxides, n-decane-1,2-oxides and indene oxides;
b) conversion of a racemate of styrene oxide with an enantioselectivity $E \geq 2$ to give (S)-phenyl-1,2-ethanol.

**14.** The use according to claim 12 or 13, the epoxide hydrolase being obtained from bacteria of the genus Streptomyces, in particular from the species S. griseus, S. thermovulgaris, S. antibioticus, S. arenae and S. fradiae, preferably from the strains Streptomyces griseus (DSM 13447), Streptomyces thermovulgaris (DSM 13448), Streptomyces arenae Tü495 (DSM 12134), Streptomyces antibioticus Tü4 (DSM 12925) or Streptomyces fradiae Tü 27 (DSM 12131).

## Revendications

**1.** Procédé de séparation de mélanges énantiomériques d'époxyde, **caractérisé en ce que** :

a) un mélange énantiomérique d'époxyde, qui comprend un substrat d'hydrolase époxyde, est incubé avec un micro-organisme, produisant une hydrolase époxyde (E.C. 3.3.2.3), du genre Streptomyces sp., un homogénat de celui-ci contenant de l'hydrolase époxyde, ou une fraction de cet homogénat ;
b) le mélange énantiomérique est amené à réagir, de préférence jusqu'à ajustement de l'équilibre réactionnel ; et
c) le mélange réactionnel est séparé.

**2.** Procédé selon la revendication 1, **caractérisé en ce qu'**un mélange énantiomérique d'un époxyde, sélectionné parmi des oxydes de styrène, des 3-phénylglycidates, des 1,2-oxydes d'hexane, des 1,2-oxydes de décane et des oxydes d'indène, est amené à réagir.

**3.** Procédé selon la revendication 1 ou 2, dans lequel le micro-organisme est une bactérie du genre Streptomyces sp., en particulier de la variété S. griseus, S. thermovulgaris, S. antibioticus, S. arenæ et S. fradiæ, de préférence issue des souches Streptomyces griseus (DSM 13 447), Streptomyces thermovulgaris (DSM 13 448), Streptomyces arenæ Tü 495 (DSM 12 134), Streptomyces antibioticus Tü 4 (DSM 12 925) ou Streptomyces fradiæ Tü 27 (DSM 12 131).

**4.** Procédé selon l'une quelconque des revendications 1 à 3, dans lequel l'hydrolase époxyde est **caractérisé par** au moins une des propriétés suivantes :

a) ouverture d'époxyde hydrolytique d'un oxyde de styrène, et d'au moins un autre composé sélectionné parmi des 3-phénylglycidates d'éthyle, des 1,2-oxydes de n-hexane, des 1,2-oxydes de n-décane et des oxydes d'indène ;
b) réaction d'un racémique d'oxyde de styrène ayant une énantiosélectivité $E \geq 2$ pour donner du (S)-phényl-1,2-éthanol.

**5.** Procédé de détection d'hydrolase époxyde, dans lequel :

a) un analyte, dans lequel une activité d'hydrolase époxyde est supposée, est incubé dans des conditions réactionnelles avec un substrat, contenant de l'époxyde, pour l'hydrolase époxyde, l'analyte étant une bactérie du genre Streptomyces sp., un homogénat de celle-ci ou une fraction de cet homogénat ;
b) une réaction colorée est mise en oeuvre avec de l'époxyde non réagi en présence de 4-nitrobenzylpyridine (NBP) ; et
c) la formulation de l'étape b) est analysée quant à la baisse de la concentration en colorant, relativement à une formulation de contrôle exempte d'hydrolase époxyde.

**6.** Procédé selon la revendication 5, **caractérisé en ce que** la baisse relative de la concentration en colorant est déterminée de manière quantitative et l'activité d'hydrolase époxyde dans l'analyte en est déduite.

**7.** Procédé selon l'une quelconque des revendications 5 et 6, **caractérisé en ce que** le substrat contenant de l'époxyde est de l'oxyde de styrène, du 3-phénylglycidate, du 1,2-oxyde d'hexane et/ou de l'oxyde d'indène sous une forme énantiomériquement pure ou un mélange énantiomérique de ceux-ci.

**8.** Procédé de criblage pour la détection de micro-organismes ayant une activité d'hydrolase époxyde et/ou ayant une aptitude à l'hydrolyse énantiosélective d'époxydes, comprenant un procédé de détection selon l'une quelconque des revendications 5 à 7.

**9.** Utilisation d'un micro-organisme du genre Streptomyces sp., d'un homogénat de celui-ci, contenant de l'hydrolase époxyde, ou d'une fraction de cet homogénat pour l'hydrolyse énantiosélective d'époxydes.

**10.** Utilisation d'un micro-organisme du genre Streptomyces sp., d'un homogénat de celui-ci contenant de l'hydrolase époxyde, ou d'une fraction de cet homogénat pour la préparation énantiosélective d'hydroxydes à partir des époxydes correspondants.

**11.** Procédé d'obtention d'une hydrolase époxyde (E.C. 3.3.2.3) contenant une fraction protéique, **caractérisé en ce que** :

a) un homogénat cellulaire est préparé à partir d'une culture d'un micro-organisme du genre Streptomyces sp. ;
b) l'homogénat est fractionné, les fractions obtenues étant testées quant à l'activité d'hydrolase époxyde, éventuellement en utilisant un procédé de détection selon l'une quelconque des revendications 5 à 7 ; et
c) les fractions ayant une activité d'hydrolase époxyde sont réunies et éventuellement encore fractionnées.

**12.** Utilisation d'un micro-organisme du genre Streptomyces sp. pour la production d'une hydrolase époxyde (E.C. 3.3.2.3).

**13.** Utilisation selon la revendication 12, dans laquelle l'hydrolase époxyde est **caractérisé par** au moins une des propriétés suivantes :

a) ouverture d'époxyde hydrolytique d'un oxyde de styrène, et d'au moins un autre composé sélectionné parmi des 3-phénylglycidates d'éthyle, des 1,2-oxydes de n-hexane, des 1,2-oxydes de n-décane et des oxydes d'indène ;
b) réaction d'un racémique d'oxyde de styrène ayant une énantiosélectivité $E \geq 2$ pour donner du (S)-phényl-1,2-éthanol.

**14.** Utilisation selon la revendication 12 ou 13, dans laquelle l'hydrolase époxyde est produit à partir de bactéries du genre Streptomyces sp., en particulier de la variété S. griseus, S. thermovulgaris, S. antibioticus, S. arenæ et S. fradiæ, de préférence à partir des souches Streptomyces griseus (DSM 13 447), Streptomyces thermovulgaris (DSM 13 448), Streptomyces arenæ Tü 495 (DSM 12 134), Streptomyces antibioticus Tü 4 (DSM 12 925) ou Streptomyces fradiæ Tü 27 (DSM 12 131).

# Fig.1

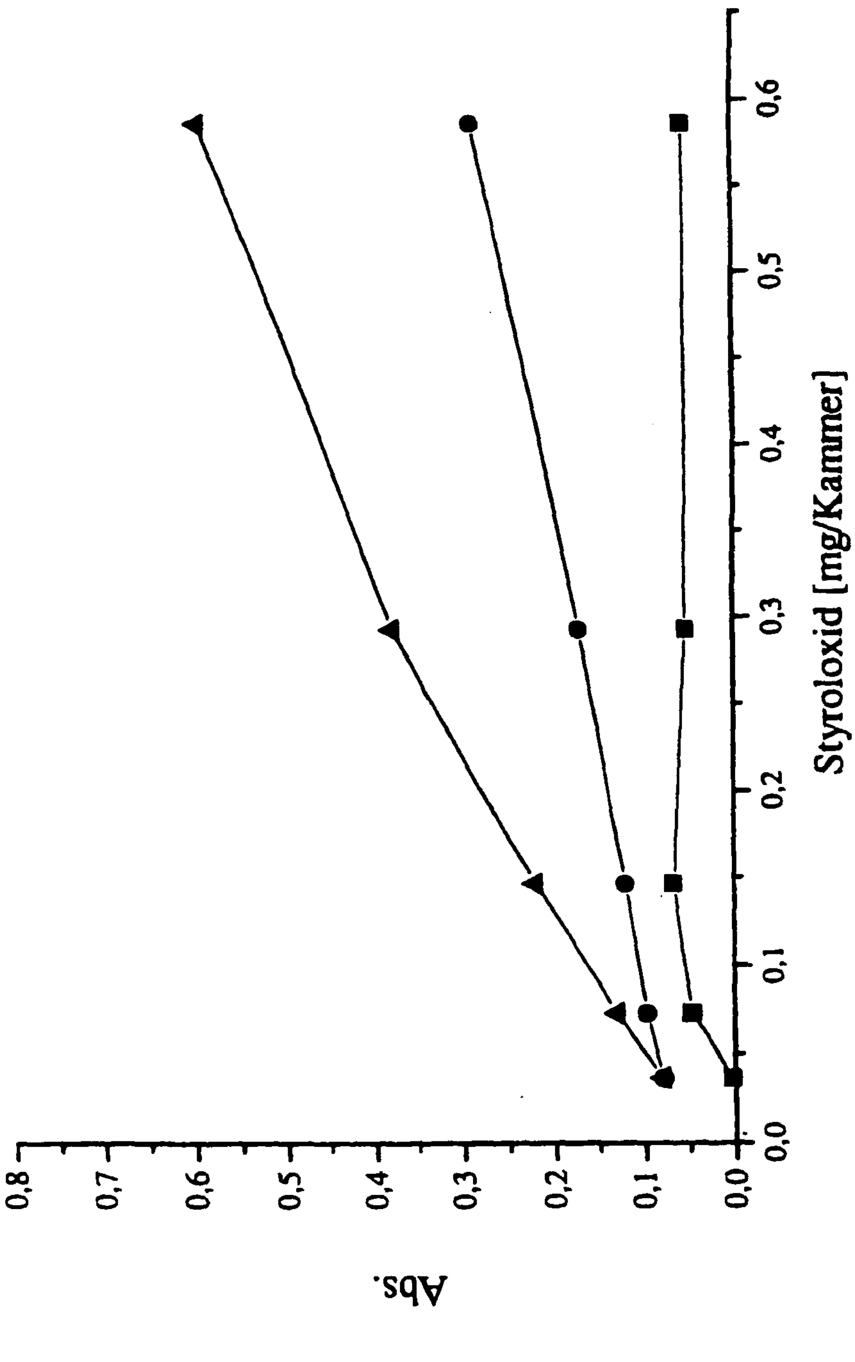
Abs.
0,0
0,1
0,2
0,3
0,4
0,5
0,6
0,7
0,8
Styroloxid [mg/Kammer]
0,0
0,1
0,2
0,3
0,4
0,5
0,6

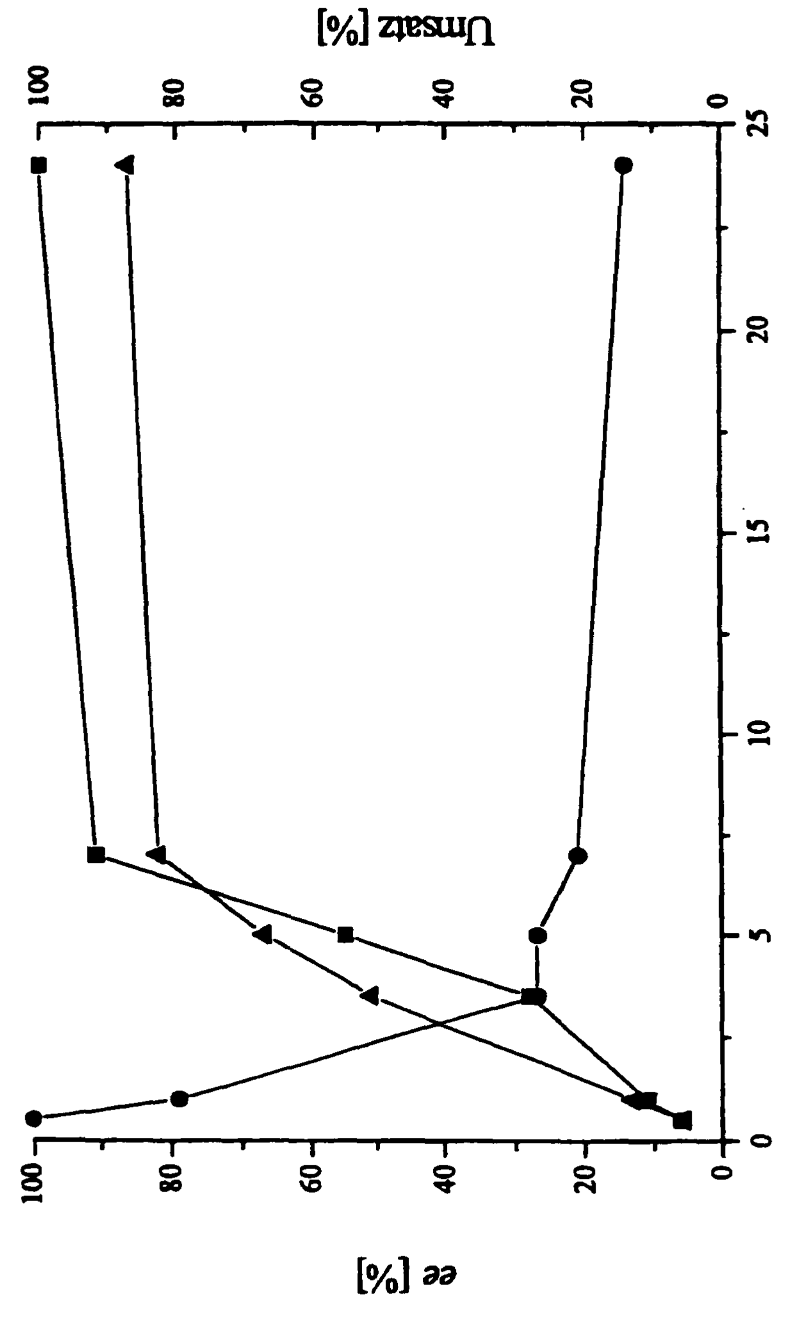


**Fig. 2**

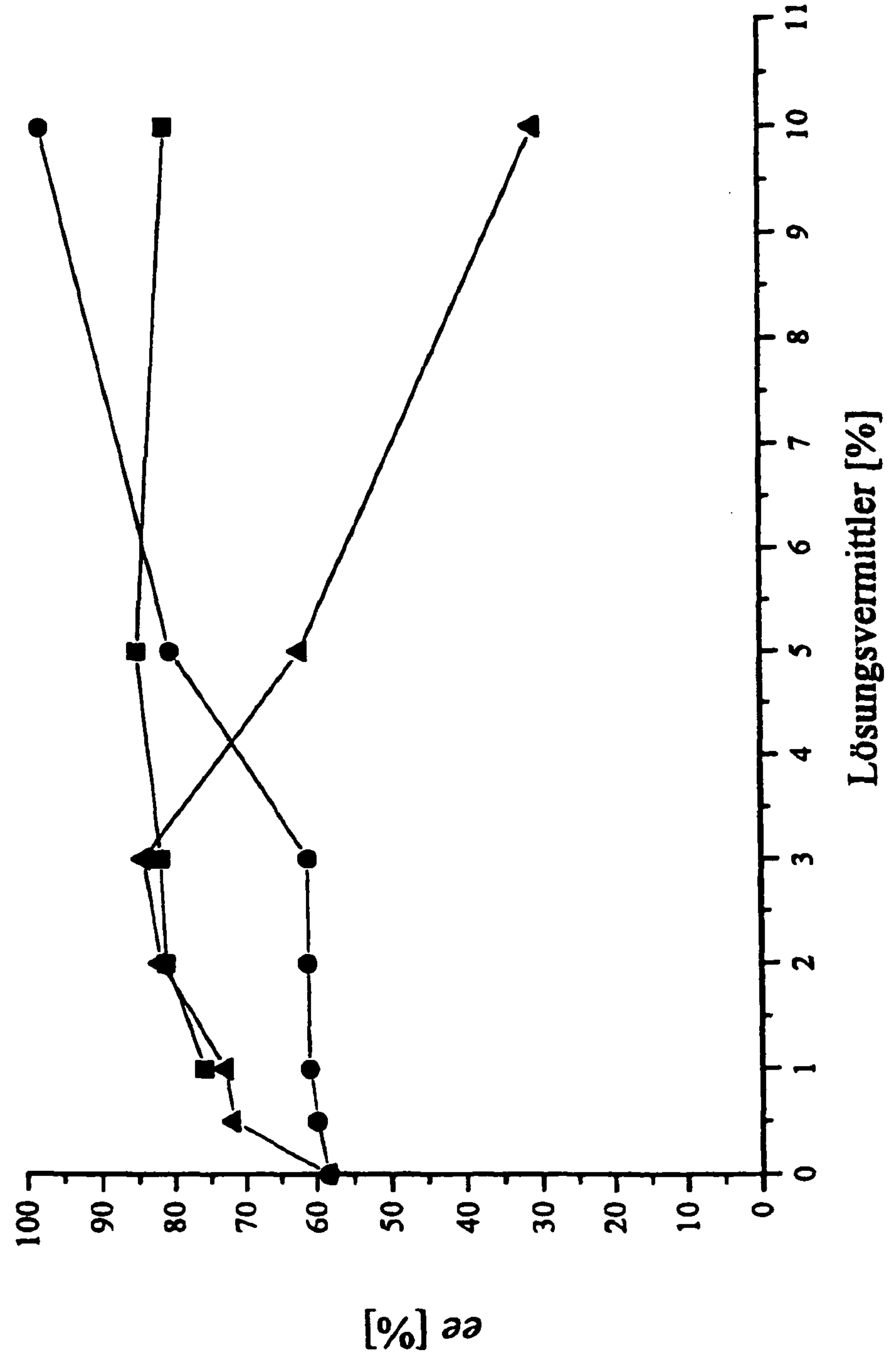
ee [%]
100
90
80
70
60
50
40
30
20
10
0
0
1
2
3
4
5
6
7
8
9
10
11
Lösungsvermittler [%]

Fig. 3

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **GROGAN, G. et al.** *FEMS Microbiology Lett.,* 1986, vol. 141, 239-243
- **KROUTIL, W. et al.** *Tetrahedron Lett.,* 1996, 8379-8382
- **MISAWA, E. et al.** *Eur. J. Biochem.,* 1998, vol. 253, 173-183
- **RINK, R. et al.** *J.Biol.Chem.,* 1997, vol. 272, 14650-14657
- **FABER, K. et al.** *Biotechnology Lett.,* 1995, vol. 17, 893-898
- **KROUTIL, W. et al.** *J. Biotechnol.,* 1998, vol. 61, 143-150
- **VON MISAWA, E. et al.** *Eur. J. Biochem.,* 1998, vol. 253, 173-183
- **CHEN, C.S. et al.** *J.Am.Chem.Soc.,* 1982, vol. 104, 7294